# EUROPEAN PATENT APPLICATION

(11) **EP 4 809 934 A1**
(43) Date of publication of application: **23.09.2026**
(21) Application number: 26164173.2
(22) Date of filing: 12.03.2026
(51) Int. Cl.: A61B 8/08, A61B 8/12, A61B 8/00

(54) **ULTRASOUND IMAGING CATHETER GUIDED PULSE FIELD ABLATION**

(30) Priority: 17.03.2025 US 202519081368
(71) Applicant: Siemens Medical Solutions USA, Inc., Malvern, PA 19355-1406 (US)
(72) Inventor: ZHANG, Yue, Princeton, NJ, 08540-6632 (US); FUNKA-LEA, Gareth, Princeton, NJ, 08540-6632 (US); KIM, Young-Ho, Princeton, NJ, 08540-6632 (US); SHARMA, Puneet, Princeton, NJ,, 08540-6632 (US)
(74) Representative: HKW Intellectual Property PartG mbB

(57) **Abstract**

Pulse field ablation (PFA) guidance is generated from intracardiac echocardiogram (ICE) imaging. The relative position of the PFA device (450) and the tissue is determined. The treatment placement with respect to the tissue for repetitive application is detected. By finding the relative position and/or treatment placement over time, ICE imaging in combination with image processing may provide visual guidance rather than just imaging or just catheter tracking for PFA. The resulting PFA procedure may be more likely complete and/or optimized.

## Description

### BACKGROUND

The present embodiments relate to ultrasound imaging with a catheter, such as an intracardiac echo (ICE) catheter. The ICE catheter may image treatment.

Catheter ablation (CA) is commonly performed for the management of atrial fibrillation (AFib). Traditional CA procedures employ a catheter to deliver thermal energy, either through radiofrequency heat or cryotherapy cold, to create controlled scars in the heart tissue. This technique typically targets the pulmonary veins and occasionally the posterior wall of the left atrium, aiming to electrically isolate these structures and prevent abnormal electrical signals. Recently, pulse field ablation (PFA) has emerged as a promising technique for the treatment of AFib. Unlike traditional thermal energy methods, PFA relies on electroporation, which uses electric fields to create microscopic pores in cell membranes.

PFA can be monitored, such as with intracardiac echocardiogram (ICE), fluoroscopy, or electro anatomic mapping. For example, PFA for pulmonary vein isolation (PVI) is monitored with two- or three-dimensional ICE guidance. The PFA procedure involves several steps. First, a transseptal puncture is performed, and an ICE imaging probe is deployed to the target position (usually in left atrium). Second, the PFA catheter is advanced into the left atrium, proximal to the ostium of the left superior pulmonary vein (LSPV). The PFA catheter then transforms into its treatment shape. Depending on the manufacturer, the treatment shape have a 'flower,' 'basket,' or 'circular' shape. These catheters have identifiable electrodes visible through imaging. Third, once transformed, the PFA catheter contacts the ostium of the target vein and delivers ultra-rapid electrical energy. The PFA catheter is then rotated and re-applied to the ostium to ensure uniform application due to the spacing between the electrodes. Finally, the PFA catheter is deployed to the remaining three veins: the left inferior pulmonary vein (LIPV), right inferior pulmonary vein (RIPV), and right superior pulmonary vein (RSPV), following the same procedure. ICE is used to visually monitor the PFA catheter by the physician.

ICE have been used to monitor other interventional procedures. Image processing is used to assist the monitoring by detecting and tracking the other catheter. However, these tracking techniques are standalone methods that focus on the treatment device itself and do not account for the specific characteristics and requirements of the PFA procedure.

### SUMMARY

The invention is defined by the attached set of claims. Further details of the disclosed method, devices and system are described below, which are helpful for understanding the claimed invention.

By way of introduction, the preferred embodiments described below include methods, systems, non-transitory computer readable media, and improvements for guiding PFA with ultrasound imaging. PFA guidance is generated from ICE imaging. The relative position of the PFA device and the tissue is determined. The treatment placement with respect to the tissue for repetitive application is detected. By finding the relative position and/or treatment placement over time, ICE imaging in combination with image processing may provide visual guidance rather than just imaging or just catheter tracking for PFA. The resulting PFA procedure may be more likely complete and/or optimized.

In a first aspect, a method is provided for guiding pulse field ablation with ultrasound imaging. Placement of a pulse field ablation electrodes at tissue of a patient is imaged with an intracardiac echocardiogram catheter. Position information for the pulse field ablation device relative to the tissue is detected from the imaging. A visual cue for alignment of the pulse field ablation device with the tissue is displayed. The visual cue based on the position information.

In a second aspect, an ultrasound system is provided for pulse field ablation guidance. An intracardiac echocardiogram catheter with a transducer is configured for ultrasound scanning from within a cardiac system of a patient. An image processor is configured to generate guidance of pulse field ablation from the ultrasound scanning. A display is configured to display the guidance.

In a third aspect, a method is provided for guiding pulse field ablation with ultrasound imaging. A pulse field ablation catheter and an ostium of a vein of a patient are scanned with an intracardiac echocardiogram catheter. Visual guidance of pulse field ablation catheter deployment in pulmonary vein isolation treatment is displayed. The visual guidance is generated from the scanning.

Any one or more of the aspects or concepts summarized above or in the Illustrative Embodiments below may be used alone or in combination. In particular, the system according to the second aspect may be used to implement the methods according to the first and/or third aspect. The aspects or concepts described for one Illustrative Embodiment or aspect may be used in other embodiments or aspects. The aspects or concepts described for a method or system may be used in others of a system, method, computer program, or non-transitory computer readable storage medium.

The present invention is defined by the following claims, and nothing in this section should be taken as a limitation on those claims. Further aspects and advantages of the invention are discussed below in conjunction with the preferred embodiments and may be later claimed independently or in combination.

### BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.
Figure 1 a flow chart diagram of one embodiment of a method for guiding PFA with ultrasound imaging;
Figure 2 illustrates a workflow for generating PFA guidance from ultrasound scanning;
Figures 3A and 3B are example ultrasound two-dimensional images showing out-of-plane placement of the PFA device;
Figure 4 is a block diagram of one embodiment of an ultrasound system for PFA guidance; and
Figure 5 illustrates an example neural network.

### DETAILED DESCRIPTION OF THE DRAWINGS AND PRESENTLY PREFERRED EMBODIMENTS

Ensuring the correct positioning of the PFA catheter relative to the pulmonary vein is useful for successful treatment, as correct placement reduces the likelihood of needing a second ablation procedure and lowers the risk of stroke. Correct placement involves two components: first, maximizing the overlap between the electrodes and the ostium during each ablation; and second, ensuring a uniform application of the ablation across the entire ostium during a given ablation. Smart features are generated to address one or more of these requirements.

Visual guidance for PFA catheter deployment is provided. The examples used herein are for pulmonary vein isolation treatment using two- or three-dimensional (2D or 3D) ICE. The visual guidance provides more information than mere ultrasound imaging of the PFA and tissue. The ablation device and the closest target pulmonary vein are simultaneously detected and tracked, displaying visual cues on the PFA device and ostium centerline in images to help the user achieve optimal alignment. During device rotation and translation, the rotational angle and/or shift from the ostium center is detected and tracked, ensuring uniform ablation treatment over the target vein.

There are currently no smart image-based features or computer-assisted solutions available for PFA device enhancement. Assurance of correct catheter contact is currently based solely on visual judgment using ICE imaging and the physician's expertise, or on electroanatomic mapping, which is static and error prone as it does not account for heart motion. While detecting and tracking of the PFA catheter alone may be integrated into a PFA device imaging, the interaction between the PFA device and target is important. By determining the optimal alignment of the PFA catheter with the target and ensuring uniform application of the treatment to the target as added visual guidance, PFA treatment is improved.

In one approach for ensuring the success of PFA treatment, an artificial intelligence (AI) is used to generate the visual guidance for PFA with ICE. Joint tracking and pose estimation of the PFA device and pulmonary vein (PV) is used for the guidance. Rather than tracking just the device itself without procedural consideration, the device relative to the target is tracked for PFA. The success criteria of PFA catheter positioning, including orientation and distance relative to PV ostium, is detected. The rotational angle estimation of PFA device to the PV ostium is detected. The electrodes on the catheter are detected from ICE imaging, and 3D registration or 2D template-based approach is used to determine the rotation angle estimation.

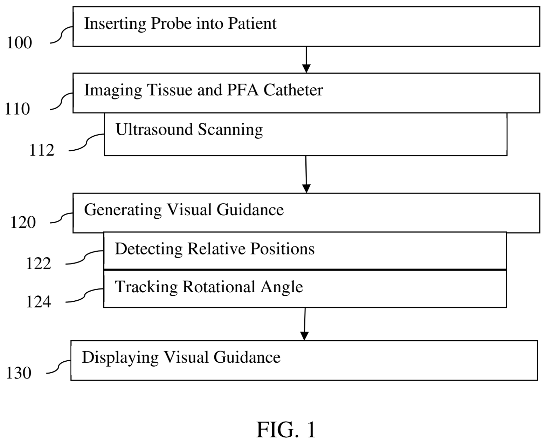

Figure 1 is a flow chart diagram of one implementation of a method for guiding PFA with ultrasound imaging. Joint catheter enhancement and pulmonary vein detection targets optimal application in PFA treatment. ICE-based imaging is used to detect both the PFA catheter and the vein, and visual guidance for optimal PFA treatment is displayed based on the detections.

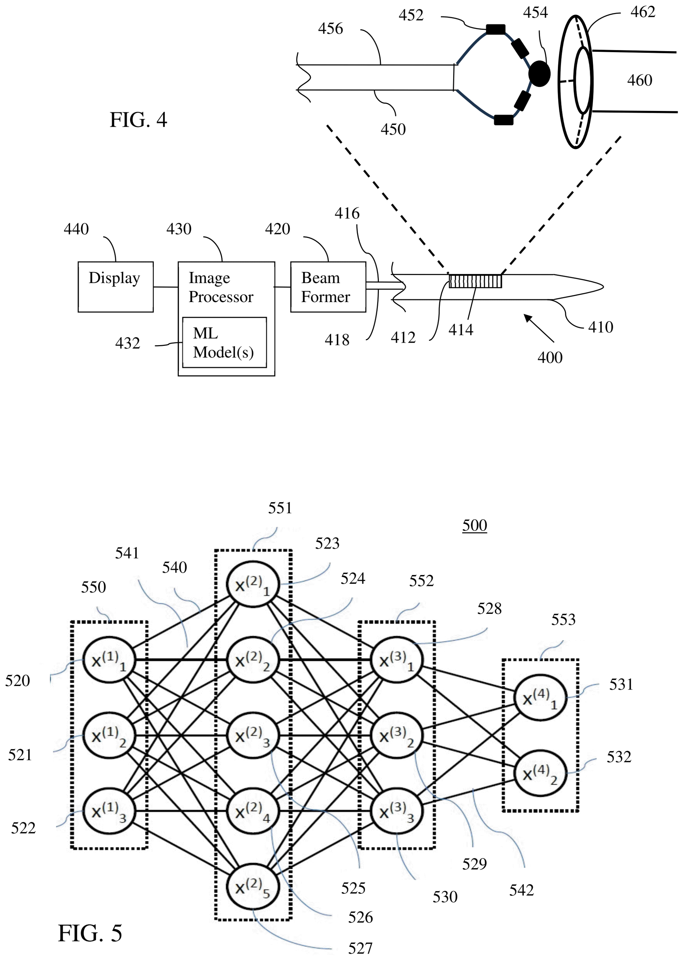

The system of Figure 4 or another system implements the acts of Figure 1. Any ultrasound system may be used. The physician performs act 100, and then the ultrasound system performs acts 110-130. A computer, workstation, server, or other image processor may perform acts 120-130.

Additional, different, or fewer acts may be provided. For example, acts for configuring the ultrasound imaging system and/or acts for diagnosis or treatment are included. As another example, acts for inserting and/or controlling both the PFA catheter and the ICE catheter are provided. In yet another example, acts 122 and/or 124 are not provided, such as where other PFA guidance than relative position or rotational angle (application placement) is provided. As another example, act 100 is not provided, such as where the method is directed to the actions or process of the ultrasound scanner.

The acts are performed in the order shown (top to bottom or numerically) or a different order. For example, acts 122 and 124 may be performed simultaneously or in an opposite order.

In act 100, the catheter probe is inserted into a patient. The probe is inserted into a lumen, such as a blood vessel. For example, the probe is a ICE catheter inserted into the cardiac system to navigate to the heart. The tip of the probe is positioned for imaging tissue of interest, such as for monitoring ablation and/or examining a vein. Guide wires, translation, and/or rotation are used to steer the probe to a position for imaging. The array of the probe is positioned so that a scan plane or volume includes the tissue of interest and at least part of the PFA catheter.

The PFA catheter is also inserted, such as after positioning the ICE catheter. The PFA catheter is steered to position a treatment part (e.g., wires, arms, head, or applicator formed as a flower, basket, circular, or another shape) by a vein.

In one implementation, a transseptal puncture is performed, and an ICE imaging probe is deployed to the target position (usually in left atrium). Second, the PFA catheter is advanced into the left atrium, proximal to the ostium of the left superior pulmonary vein (LSPV) or another vein. The PFA catheter then transforms into its treatment shape. The treatment part of the PFA catheter includes electrodes visible through ultrasound imaging. Acts 110-130 are performed to guide treatment by the PFA catheter. For example, once transformed, the PFA catheter contacts the ostium of the target vein based on the visual guidance and delivers ultra-rapid electrical energy. The PFA catheter is then rotated and re-applied to the ostium based on visual guidance to ensure uniform application due to the spacing between the electrodes. Finally, the PFA catheter may be deployed to another vein, such as each of the remaining three veins: the left inferior pulmonary vein (LIPV), right inferior pulmonary vein (RIPV), and right superior pulmonary vein (RSPV). The same procedure using the visual guidance output in act 130 is performed for each of the veins.

In act 110, the ultrasound system images the tissue to be treated and the PFA catheter. An imaging array of the ICE catheter images the patient and PFA catheter. The ultrasound scanner images in the plane defined by the imaging array. Volume imaging may be provided using a multi-dimensional array, shaped 1D array, or movement of the 1D array.

In act 112, the array connects with the beamformer to scan the patient and PFA catheter. The scan region is scanned with ultrasound, and an ultrasound image or images of tissue and/or fluid of the patient is generated in act 110. Any imaging (e.g., B-mode and/or flow or color mode) may be used. The imaging generates one or more images, such as images representing tissue and the PFA catheter in a two or three-dimensional region. The images may be image frames of scan data prior to scan conversion or after scan conversion.

The imaging, as well as operation of the PFA catheter, may be gated to the cardiac cycle. To limit motion effects, the imaging and/or ablation is performed during a same phase of the cardiac cycle. Motion compensation in imaging or scanning may be used. One or both catheters may be anchored to move with the heart tissue.

The placement of the PFA treatment part and/or the electrodes at tissue of a patient are imaged with the ICE catheter. For example, the PFA electrodes as placed at an ostium of a vein are imaged. The imaging may be for guiding the treatment part to the ostium and/or for guiding repetitive placements to treat the ostium more thoroughly.

In act 120, an image processor, such as a processor of the ultrasound scanner, generates visual guidance. The visual guidance is generated from detection of the pulse field ablation catheter and the ostium. The visual guidance indicates the PFA catheter deployment relative to the ostium. The visual guidance is a cue (e.g., an enhancement or information) more than a mere image of the tissue and PFA treatment part. The visual guidance is added highlighting, graphic, annotation, or other information generated through image processing to add visual information, such as adding to the ultrasound images of the tissue and PFA catheter.

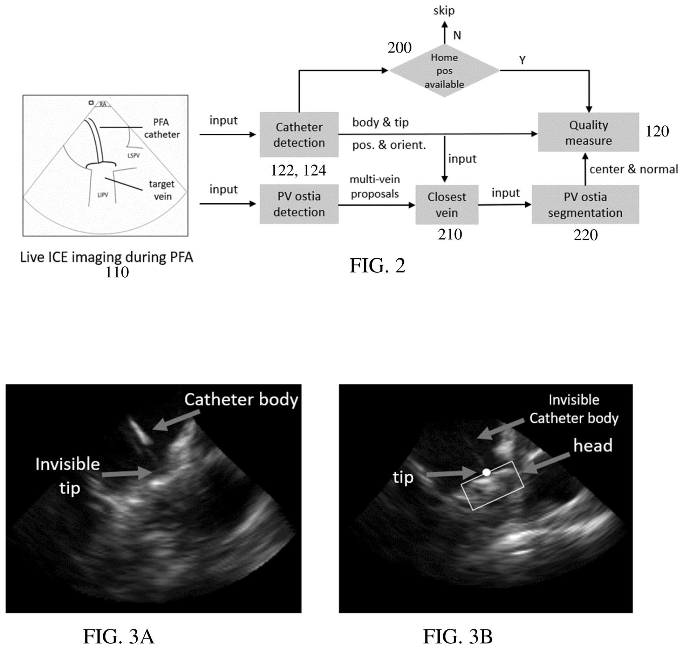

Figure 2 shows an example approach for generating the visual guidance. The PFA device and target vein are both detected and tracked in act 122 and/or act 124 from live ICE imaging 110. For the PFA catheter, the body, treatment tip, and/or electrodes are detected. Multiple veins or vein regions may be detected, such as pulmonary vein ostia. Where multiple veins are in the imaging, a closest one is selected in act 210. For the selected vein, the ostium is detected or segmented in act 220. Where the rotation angle is to be determined for mapping electrode placements on the ostium, a home position (e.g., first treatment position or rotation angle) may be defined in act 200. The treatment is repeated for different rotational angles (i.e., electrodes at different positions on ostium) based on the home position. In act 120, one or more quality measures continuously assessing device positioning metrics, such as relative position and orientation between ablation catheter and PV ostia center and rotational angle and shift of device against a pre-set home position, are generated. Such PFA treatment quality measurements are displayed to the user via a visualization module. Other information may be displayed, such as highlighting or enhancement (e.g., greater resolution or filtered differently) of the objects detected in act 122 and/or act 124 in the ultrasound images.

Act 122 and act 124 are two examples of detecting for specific quality measurements to be used as visual guidance. Additional, different, or fewer of these acts for PFA treatment guidance may be performed. Other tissue and/or PFA catheter detection may be performed to provide another indicator of or to assist in quality PFA application.

In act 122, the relative position of the treatment part to the ostium is detected. By detecting the position of the ostium and treatment part, the angle of the PFA catheter (shaft) and distance of the treatment part to the vein and/or ostium may be measured. This position information allows for proper alignment of the treatment part to the ostium. For example, a 90 degree or perpendicular angle of the shaft of the PFA catheter to a normal from a plane through the ostium with a given distance (e.g., treatment part tip within the vein (e.g., negative distance)) indicates proper positioning. The angle and/or distance generated from the detected relative positions guides the user to properly position the treatment part on or against the ostium.

In act 124, the rotational angle of the treatment part to the ostium is detected. The rotation angle is for rotation in the plane of the ostium. By detecting the positions of electrodes and/or rotational angle of the PFA catheter relative to the ostium, the locations of treatment on the ostium are determined. Since the treatment part is rotated relative to the ostium and re-applied to cover additional points on the ostium, the rotational angle may be tracked. The rotational angle or the electrode placements on the ostium due to the rotation are tracked as the rotational angle. The rotational angle for the different or series of ablations by the electrodes on the tissue is detected and/or tracked. A visual cue of the rotational angle, such as a map of the rotational angles of different ablations, guides the user to better cover the ostium form ablation.

Different detection is performed for each type of object (e.g., electrodes, PFA shaft by the treatment head, treatment tip, vein, and/or ostium). Alternatively, one process is used to detect multiple types of objects.

The PFA catheter and tissue are detected using image processing. For example, template matching, random walker, other landmark or object detection, and/or other segmentation is performed. Alternatively, or additionally, an artificial intelligence (AI) detects. The ultrasound image(s) are input to a machine-learned model, which outputs the detection (e.g., the position in the image).

For AI, the detection is performed with a machine-learned model, such as a machine-learned neural network. The machine-learned model may be any now known or later develop machine-trained model, such as a Bayesian network, neural network, or a support vector machine. In one implementation, the machine-learned model is a neural network trained with deep learning.

The model architecture is arranged to receive the original image or sequence of images (image data). The machine-learned network is configured to output the object in response to input of the imaging data. The machine-learned network is a fully connected, convolutional, or another neural network. Any network structure may be used. Any number of layers, nodes within layers, types of nodes (activations), types of layers, interconnections, learnable parameters, and/or other network architectures may be used. In one approach, the neural network is configured as an image-to-image network, such as an encoder-decoder or U-Net.

Machine training uses the defined architecture, training data, and optimization to learn values of learnable parameters of the architecture based on the samples and ground truth of training data. For training the model to be applied as a machine-learned model, training data is acquired and stored in a database or memory. The training data is acquired by expert review, aggregation, mining, loading from a publicly or privately formed collection, transfer, and/or access, such as collecting from patient medical records. Ten, hundreds, or thousands of samples of training data are acquired. The samples are from scans of different patients and/or phantoms. Simulation may be used to form the training data. The training data includes many samples of the desired output (ground truth), such as detected objects, and the input, such as imaging data. In one implementation, a large dataset of ICE images, each annotated with the PFA device in the field of view, is used to train. Optionally, a 3D model of the catheter can be used as another input for the deep learning and resulting trained model to provide shape prior information when available. Optionally, the training database includes synthetically generated ICE images where the device is introduced in the image field of view.

A machine (e.g., image processor, server, workstation, or computer) machine trains the neural network or other machine learning model to detect the object. The training uses the training data to learn values for the learnable parameters (e.g., convolution kernels, node weights, link weights, and/or settings of activation functions) of the network. The training determines the values of the learnable parameters of the network that most consistently output close to or at the ground truth given the input samples. In training, the loss function may be L1 between predicted objects and ground truth objects. Other loss functions, such as cross entropy or L2, may be used. Adam, gradient descent, another first order optimization algorithm, or another function is used for optimization (e.g., to minimize the loss or maximize a gain).

Once trained, the machine-learned or trained neural network is stored for later application. The training determines the values of the learnable parameters of the network. The network architecture, values of non-learnable parameters, and values of the learnable parameters are stored as the machine-learned network. Copies may be distributed, such as to ultrasound scanners, for application. Once stored, the machine-learned network may be fixed. The same machine-learned network may be applied to different patients, different scanners, and/or different treatments.

The machine-learned network may be updated. As additional training data is acquired, such as through application of the network for patients and corrections by experts to that output, the additional training data may be used to re-train or update the training.

In another approach, the AI is a multi-task neural network. Multiple outputs are defined in the architecture. For example, an encoder-decoder arrangement is provided where there are multiple decoders inferring from the bottleneck features generated by the encoder. The encoder receives the ultrasound image or images, and the different decoders infer the different outputs from the encoded features generated by the encoder. In training, the loss is a weighted average or other combination of losses from the differences between the ground truth outputs and the predicted outputs.

For example, the multi-task neural network is configured by design and training to output both a catheter body (e.g., shaft by the treatment part) and treatment tip of the PFA device. The locations or detection of the catheter body is one task or output, and the location or detection of the treatment tip is the other task or output for a same input image. The multi-task neural network detects both the catheter body and the treatment tip for a given time (image).

In one approach, the PFA catheter is automatically detected and tracked over time. Given a live stream of ICE imaging acquired during PFA, the AI identifies and tracks the PFA catheter's body and ablation head in real-time. The output is the segmentation of the catheter body and the location of the catheter tip (e.g., tip of the treatment head), and, optionally, the electrodes of the ablation device.

If the ICE imaging used is 3D ICE, an example deep learning architecture may be a 3D UNet with multi-task decoders. The network takes a 3D ICE volume (and optionally 3D model of the catheter if known as a priori) as input, and outputs a segmentation map of the catheter body in one decoder and outputs a heatmap of a same size, where each pixel or voxel value indicates the probability of the candidate position of the catheter tip. The location with highest heat indicates the predicted tip location. A tracking by detection technique can be applied to continuously track the catheter and its tip, where the UNet is applied on each individual frame of the 3D ICE. Optionally, a post-processing technique, such as Kalman filtering, can be applied on the segmentation mask and tip location to ensure a smooth trajectory of the predicted results over time.

If the ICE imaging used is 2D ICE, an example deep learning architecture may be a 2D UNet with multi-task decoders. The decoders output the segmentation of the catheter body and prediction of the catheter tip in 2D. Compared with 3D ICE, an additional challenge for 2D ICE is that the catheter (which is in 3D space) may have out-of-plane translation, causing partial visibility on the image. Figure 3A shows an example where the tip is out-of-plane, but the body is not. Figure 3B shows an example where the body is out-of-plane, but the tip is not. The AI or image processing may output a binary score indicating whether the device is aligned with the ICE image view. For example, if the one of the decoder outputs is empty (i.e., the network does not provide catheter segmentation or the tip location), the binary score is set to be 0. Visual guidance is generated to indicate that the user should manipulate the ICE catheter to correct pose, so the PFA catheter is in-plane. If non-empty results are provided from the decoders on both body and tip, the distance between the two is calculated. If the distance is not 0 (i.e., the tip is not on the body, which violates a physical constraint), the score is set as 0. Visual guidance is generated to indicate that the user should correct catheter pose to be in-plane. Where the score is 1, the locations of both the PFA body and tip are provided.

In one approach, the closest pulmonary vein ostium is automatically detected and tracked over time. In parallel with catheter detection and tracking, the target vein, including both structure detection and motion tracking, is employed. Multiple machine-learned models may be used. In a first stage, a closest PV to the PFA treatment part is detected. One machine-learned model is configured to detect all or multiple pulmonary veins in the ICE field of view. The PV ostia are differentiated from surrounding cardiac structures. For example, the machine-learned model outputs coarse descriptors of the target, such as bounding boxes around the ostia of the PVs. Precise delineation of the PVs is not needed. The goal of this stage is to localize the target veins and corresponding regions of intertest (ROI). Upon receiving the detected catheter tip position, the closest PV to the PFA treatment tip is found based on distance of the tip location to the ostia/box centers. In other approaches, one machine-learned model outputs the detected, closest PV in one stage.

In a next stage, another machine-learned model detects (e.g., segments) and tracks the PV ostium with finer details in the localized ROI. The position of the ostium is continuously tracked, providing real-time updates to the operator. By accurately identifying the ostium and outputting visual guidance (e.g., highlighting) indicating the ostium location, the physician or operator is assisted in aligning the PFA catheter with the target vein, facilitating precise and effective ablation.

If the ICE imaging used is 3D ICE, a 3D UNet may be trained and used to produce 3D segmentation of the PV ostium. For processing efficiency (real-time segmentation), the UNet may run on a locally cropped ROI around the coarse descriptor received from the previous stage. If the ICE imaging used is 2D ICE, a 2D UNet may be trained and used to produce PV ostium landmark detection. As 2D ICE displays a cross-section of the PV ostium, two points on the PV ostium will be visible in the field of view, assuming the ostium is not parallel due to tracking the PFA device. The 2D UNet may have two decoders, with each decoding a heatmap indicating the location of an ostium point (intersection of the ostium with the ultrasound field of view).

A tracking by detection technique is applied to continuously track the PV ostium, where the UNet is applied on each individual image frame. Optionally, a post-processing technique, such as Kalman filtering, is applied on the 3D ostium segmentation or ostium points to ensure a smooth trajectory of the predicted results over time.

The detected objects (e.g., PFA shaft, PFA treatment tip, and ostium) are used to determine one or more relative positions for visual guidance. The image processor calculates the angle of the shaft to the ostium using the tracked segmentation of catheter body and a PV surface normal direction. A centerline is fit to the catheter body segmentation mask. If using 3D ICE, a 3D plane may be fit to the detected ostium points, and the normal of the 3D plane is used for angle calculation. A zero angle is desired for this arrangement. A perpendicular angle is desired where the angle is of the shaft to a plane fit to the ostium. If using 2D ICE, the normal of the ostium can be approximated by the normal to the ostium connection. The desired angle is zero or perpendicular for PFA treatment so that the treatment part rests around the ostium with generally equal force around the ostium.

The image processor calculates the distance between the tracked tip location and the center of PV ostium. The distance may be displayed as visual guidance as a positive number if the catheter treatment tip is detected exterior of PV and negative if the treatment tip is inserted past the ostium interiorly in the vein. A negative number may be desired in this arrangement so that the treatment part is pressed to the ostium. Other distances may be used, such as the distance of the end of the shaft or of electrodes to the ostium. The desired distance is established based on the measurement.

For generating visual guidance for rotational angle, the image processor detects one or more (e.g., all) the electrodes of the PFA device. A machine-learned model and/or template (e.g., physics model) matching may be used to detect the electrodes. Real-time detection and display of the PFA catheter rotation is performed. Once the user sets the initial angle (i.e., home position) as the treatment part positioned against the ostium, the rotation of the catheter (treatment part) as it is maneuvered for subsequent ablation of the same ostium is tracked. The rotational movement of the catheter is continuously tracked for display of the rotated angles relative to the starting pose and/or ostium. This quality measurement is particularly useful during the ablation procedure, as measurement ensures that the catheter maintains optimal contact with the PV ostium throughout the rotation and coverage of the ostium by rotation.

If the ICE imaging used is 3D ICE, a landmark detection algorithm detects electrodes on the catheter head as 3D point clouds. For example, a machine-learned model with an architecture and training based on deep reinforcement learning is used. Other machine-learned models, such as UNet, may be used. The machine-learned model is used on each image frame to continuously track the electrodes. Likely, during such rotation manipulation, the treatment part (head) will only have rigid motion (with small deformation due to the surface of ostium not being a perfect circle). A rigid registration between the tracked electrodes cloud and the initial electrodes cloud in a pre-set home pose is performed, and thus the rotation angle can be determined. Alternatively, the machine-learned model directly outputs the rotation angle.

If the ICE imaging used is 2D ICE, the rotation angle is at an out-plane angle. A dictionary-based template set may be used in this scenario. In a pre-procedure simulated virtual environment, a series of 2D template images showing the PFA catheter rotations are generated, together with its rotation angle. During the procedure, the system continuously matches the detected catheter (body, tip, and electrodes) with the closest template, and then uses the angles between the matched templates to estimate the rotation angle.

In act 130, the image processor generates an image with visual guidance, and a display screen displays the visual guidance as a cue to the viewer. The visual guidance of the PFA catheter deployment in PV isolation treatment is displayed. The visual guidance is generated from the scanning of act 112 (imaging of act 110) based on the image processing used to generate the visual guidance in act 120.

For example, the visual guidance is a graphic, highlighting, enhancement, annotation, or alphanumeric text for the angle of the shaft of the PFA catheter relative to the ostium, the distance of the treatment part (e.g., tip) of the PFA catheter to the ostium, the rotational angle, a map of application locations based on the rotational angle, the ostium, part of the PFA catheter, in-plane score, and/or other quality measurement or guidance to assist with PFA.

For example, the body, tip, and score are visualized through a display module, enhancing both visibility and position accuracy. The detected and tracked catheter may be highlighted with color. The PFA catheter or detected anatomy such as the PV ostia may be shown in a higher spatial resolution or using a different reconstruction.

As another example, the angle and distance between the PFA catheter and the vein or ostium is displayed. These and/or other measurements assessing the quality of PFA catheter placement relative to the vein are displayed. Both the angle and distance between the PV ostium and the catheter are used for proper PFA catheter placement for ablation. Proper angling of the shaft to the ostium ensures the correct orientation of the catheter relative to the PV ostium, and the distance ensures the correct relative location between the two (e.g., correct or even distribution of pressure). By providing real-time feedback on these measurements, the system aids the operator in adjusting the PFA catheter's orientation and distance to maximize contact with the PV ostium, enhancing the effectiveness of the ablation.

In yet another example, a map of treatment formed from electrodes of the PFA catheter during the pulmonary vein isolation treatment with the electrodes at different rotations relative to the ostium is displayed as the visual guidance. The rotation angle as an angle or a map of electrodes relative to the ostium and/or other electrode placements is visualized on a display module. By providing visual cues on the degree of rotation, the operator is assisted in achieving uniform ablation coverage.

The visual guidance is a visual cue displayed to the operator of the PFA catheter for alignment of the PFA device with the tissue. The visual cue is based on the detected position information of the PFA catheter and/or the tissue. The visual guidance is a cue to the user for proper positioning to ablate. Any cue representing the visual guidance may be used, such as displaying the visual cue as a graphic, highlight, or annotation on ultrasound images from the imaging. The visual cue may be displayed separately from the ultrasound images.

Figure 4 shows an ultrasound system for PFA guidance. The ultrasound system is used for guiding the PFA catheter 450 relative to the ostium 462 of the vein 460. The ICE catheter 400 generates images, from which relative position of the PFA catheter 450 to the vein 460 is detected for visually guiding placement. The electrodes 452, tip 454, and/or shaft 456 may be detected.

The ultrasound imaging system includes the ICE catheter 400 (e.g., array 412 of elements 414 and a housing 410) and an ultrasound scanner (e.g., a beamformer 420, an image processor 430, and a display 440). Additional, different, or fewer components may be provided. For example, the system includes the image processor 430 and display 440 without the beamformer 420 and/or ICE catheter 400, such as where the image processor 430 operates on images formed by another device. The transducer array 412 and ICE catheter 400 releasably connect with the ultrasound scanner or imaging system.

The ICE catheter 400 includes the housing 410, the array 412 of elements 414, the conductors 416, and one or more guide wires 418. Additional, different, or fewer components may be provided. For example, a port or tube for inserting and/or withdrawing fluid from the housing 410 is included. As another example, one or more markers (fiducials) for position determination are included.

The housing 410 is a sleeve of plastic or other material for insertion into a patient. For example, the housing 410 is formed from Pebax. Other materials, such as other Nylons or biologically neutral (or biocompatible) materials, may be used. The housing 410 is sealed over the array 412 to separate fluids of the patient from the interior of the catheter 400.

The imaging array 412 is in or on the ICE catheter 400. The array 412 is a transducer configured for ultrasound scanning from within a cardiac system of a patient. The array 412 has a plurality of elements 414, electrodes, and a matching layer. Additional, different, or fewer components may be provided, such as a backing block. For example, two or more matching layers are used. As another example, a semiconductor chip (e.g., application specific integrated circuit) is stacked with the array 412 in the catheter housing 410.

The elements 414 may contain piezoelectric material. Alternatively, a microelectromechanical device, such as a flexible membrane, is used. Any now known or later developed ultrasound transducer may be used.

In one embodiment, the array 412 is a 1D array. The elements 414 are distributed along a straight or curved line to form the 1D array of elements 414. In other embodiments, the array 412 is a 1.5D or 2D array (multi-dimensional). In yet other embodiments, any array having one dimension greater than the width (diameter) of the probe body and another dimension less than the width may be used. For example, a planar imaging array produced as a capacitive micromachined ultrasound transducer (CMUT) where each element is composed of a matrix of micro-elements is used. A helical array, 2D array, or 1D array rotated to different positions may be used for 3D imaging. The 1D array in one position may be used for 2D imaging.

The array 412 is positioned distally from the steering section of the housing 410 of the probe 100. The array 412 is in or near a tip of the catheter housing 410. Other positions may be provided. The array 412 is positioned along the longitudinal axis within the housing 410.

The ultrasound scanner (e.g., beamformer 420, image processor 430, and/or display 440) is configured for ultrasound imaging. The array 412 is used to form an aperture for a scan plane. The beamformer 420 uses elements 414 of the array 412 to scan an image plane or volume. Conductors 416 connect the array 412 to the beamformer 420 for imaging. The beamformer 420 includes a plurality of channels for generating transmit waveforms and/or receiving signals.

The image processor 430 is a detector, filter, processor, application specific integrated circuit, field programmable gate array, digital signal processor, control processor, controller, scan converter, three-dimensional image processor, graphics processing unit, AI processor, tensor processor, analog circuit, digital circuit, or combinations thereof. The image processor 430 receives beamformed data and generates images on the display 440. In one implementation, the image processor 430 as one device or a combination of multiple devices generates the ultrasound image (e.g., detector and scan converter) and generates guidance for PFA form the ultrasound scanning (from scan data and/or images) (e.g., general processor).

The image processor 430 is configured by firmware, software, and/or hardware. The image processor 430 is configured to generate guidance of PFA from the ultrasound scanning. For example, the image processor 430 is configured to generate the guidance as an angle of a PFA catheter 450 relative to tissue and/or a distance of a treatment tip of the PFA catheter 450 to the tissue. Detection of the relative objects, such as the ostium 462, PFA shaft 456, electrodes 454, and PFA treatment tip 454, is performed. One or more machine-learned models 432 may be used for the detection (e.g., segmentation) of the objects.

The guidance is generated from the detected locations, such as generating distance from the treatment part 454 to the ostium 462, angle of the shaft 456 to a normal to the ostium 462, and/or rotation angle of the treatment head 454 to the ostium 462. For example, a treatment map showing the different angles and/or electrode positions of the PFA catheter 450 relative to the tissue (e.g., ostium 462) for different ablations is generated. The detection of the tissue and the PFA in the ultrasound imaging is used to generate the guidance, such as one or more quality measurements for aligning PFA to the vein 460 and applying the ablation.

The instructions for implementing the processes, methods, and/or techniques discussed herein are provided on non-transitory computer-readable storage media or memories, such as a cache, buffer, RAM, removable media, hard drive, or other computer readable storage media (e.g., memory of the ultrasound scanner). Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination.

In one embodiment, the instructions are stored on a removable media device for reading by local or remote systems. In other embodiments, the instructions are stored in a remote location for transfer through a computer network. In yet other embodiments, the instructions are stored within a given computer, CPU, GPU or system. Because some of the constituent system components and method steps depicted in the accompanying figures may be implemented in software, the actual connections between the system components (or the process steps) may differ depending upon the manner in which the present embodiments are programmed.

The display 440 is a monitor, liquid crystal display, television, tablet, mobile device, or another screen configured to display one or more images. Other displays, such as a printer, may be used. The display 440 is configured by loading an image into memory (e.g., display plane or buffer) to display the image. The display 440 is configured to display an image with guidance. For example, a sequence of ultrasound images is displayed. Each image indicates the angle of the shaft relative to the ostium, the distance of the treatment head (e.g., tip) to the ostium, and/or the rotational angle (as an angle or as a map based on a series of rotational angles) of the shaft, treatment head, and/or electrodes to the ostium. Other visual guidance for PFA may be provided, such as highlighting or enhancing particular objects in ultrasound images, such as the ostium, PFA shaft, PFA electrodes, and/or PFA treatment head. Other guidance may be used, such as indicating a desired measurement and/or indicating any deviance from the desired measurement.

The user, upon viewing the guidance, steers the PFA catheter. The guidance assists or helps with proper placement of the PFA catheter relative to the tissue. The resulting ablation more likely provides the desired effect due to the visual guidance. By providing the guidance continuously, regularly, or periodically, the continued placement of the PFA catheter relative to the vein or veins is guided over time.

Figure 5 shows an embodiment of an artificial neural network 500, in accordance with one or more embodiments. Alternative terms for "artificial neural network" are "neural network," "artificial neural net," or "neural net." The artificial neural network 500 may be used in part in, for example, the one or more machine learning based networks utilized for compounding or generating 3D deformation. The artificial neural network 500 may be arranged or designed as a neural field for ultrasound 3D compounding and/or generating a deformation field.

The artificial neural network 500 includes nodes 520-532 and edges 540-542, wherein each edge 540-542 is a directed connection from a first node 520-532 to a second node 520-532. In general, the first node 520-532 and the second node 520-532 are different nodes 520-532, it is also possible that the first node 520-532 and the second node 520-532 are identical. For example, in Figure 5, the edge 540 is a directed connection from the node 520 to the node 523, and the edge 541 is a directed connection from the node 521 to the node 523. An edge 540-542 from a first node 520-532 to a second node 520-532 is also denoted as "ingoing edge" for the second node 520-532 and as "outgoing edge" for the first node 520-532.

In this embodiment, the nodes 520-532 of the artificial neural network 500 may be arranged in layers 550-553, wherein the layers may include an intrinsic order introduced by the edges 540-542 between the nodes 520-532. In particular, edges 540-542 may exist only between neighboring layers of nodes. In the embodiment shown in Figure 5, there is an input layer 550 including only nodes 520-522 without an incoming edge, an output layer 553 including only nodes 531 and 532 without outgoing edges, and hidden layers 551, 552 in-between the input layer 550 and the output layer 553. In general, the number of hidden layers 551, 552 may be chosen arbitrarily. The number of nodes 520-522 within the input layer 550 usually relates to the number of input values of the neural network 500, and the number of nodes 531-532 within the output layer 553 usually relates to the number of output values of the neural network 500. For a neural field, the number of layers and nodes corresponds to the coordinate system. Each node may be connected to adjacent nodes in any direction in a bidirectional edge. The input may be to all the nodes with output to all the nodes.

In one approach, the network architecture is an MLP with a small number of fully-connected layers, where the input to the network is a 3D position (mapped to a higher dimension using positional encoding), and the output of the network is a vector of parameters used by a volume renderer (attenuation, reflectance, scattering at the 3D position).

A (real) number may be assigned as a value to every node 520-532 of the neural network 500. Here, x⁽ⁿ⁾ᵢ denotes the value of the i-th node 520-532 of the n-th layer 550-553. The values of the nodes 520-522 of the input layer 550 are equivalent to the input values of the neural network 500, the value of the nodes 531-532 of the output layer 553 is equivalent to the output value of the neural network 500. Furthermore, each edge 540-542 may include a weight being a real number, in particular, the weight is a real number within the interval [-1, 1] or within the interval [0, 1]. Here, w^{(m,n)}_{i,j} denotes the weight of the edge between the i-th node 520-532 of the m-th layer 550-553 and the j-th node 520-532 of the n-th layer 550-553. Furthermore, the abbreviation w⁽ⁿ⁾_{i,j} is defined for the weight w^{(n,n+1)}_{i,j}.

In particular, to calculate the output values of the neural network 500, the input values are propagated through the neural network. In particular, the values of the nodes 520-532 of the (n+1)-th layer 550-553 may be calculated based on the values of the nodes 520-532 of the n-th layer 550-553 by: ${x}_{j}^{\left(n+1\right)} = f \left({\sum }_{i} {x}_{i}^{\left(n\right)} ⋅ {w}_{i , j}^{\left(n\right)}\right) .$

Herein, the function f is a transfer function (another term is "activation function"). Known transfer functions are step functions, sigmoid function (e.g., the logistic function, the generalized logistic function, the hyperbolic tangent, the Arctangent function, the error function, the smoothstep function) or rectifier functions. The transfer function is mainly used for normalization purposes.

In particular, the values are propagated layer-wise or through adjacent nodes through the neural network 500, wherein values of the input layer 550 are given by the input of the neural network 500, wherein values of the first hidden layer 551 may be calculated based on the values of the input layer 550 of the neural network 500, wherein values of the second hidden layer 552 may be calculated based in the values of the first hidden layer 551, etc.

In order to set the values w^{(m,n)}_{i,j} for the edges, the neural network 500 has to be trained using training data. In particular, training data includes training input data and training output data (denoted as tᵢ). For a training step, the neural network 500 is applied to the training input data to generate calculated output data. In particular, the training data and the calculated output data include a number of values, said number being equal with the number of nodes of the output layer.

In particular, a comparison between the calculated output data and the training data is used to recursively adapt the weights within the neural network 500 (backpropagation algorithm). In particular, the weights are changed according to: $w {′}_{i , j}^{\left(n\right)} = {w}_{i , j}^{\left(n\right)} - γ ⋅ {δ}_{j}^{\left(n\right)} ⋅ {x}_{i}^{\left(n\right)}$ wherein γ is a learning rate, and the numbers δ⁽ⁿ⁾ⱼ may be recursively calculated as: ${δ}_{j}^{\left(n\right)} = \left({\sum }_{k} {δ}_{k}^{\left(n+1\right)} ⋅ {w}_{j , k}^{\left(n+1\right)}\right) ⋅ f ′ \left({\sum }_{i} {x}_{i}^{\left(n\right)} ⋅ {w}_{i , j}^{\left(n\right)}\right)$ based on δ⁽ⁿ⁺¹⁾ⱼ, if the (n+1)-th layer is not the output layer, and ${δ}_{j}^{\left(n\right)} = \left({x}_{k}^{\left(n+1\right)}-{t}_{j}^{\left(n+1\right)}\right) ⋅ f ′ \left({\sum }_{i} {x}_{i}^{\left(n\right)} ⋅ {w}_{i , j}^{\left(n\right)}\right)$ if the (n+1)-th layer is the output layer 553, wherein f' is the first derivative of the activation function, and y⁽ⁿ⁺¹⁾ⱼ is the comparison training value for the j-th node of the output layer 553.

Listed below are various Illustrative Embodiments. The Illustrative Embodiments summarize different combinations of aspects or features. Other combinations of any of the aspects or features with any other one or more of the aspects or features may be provided. Aspects or features from one type (e.g., method or system) may be used in another type (system or method).

Illustrative Embodiment 1. A method for guiding pulse field ablation with ultrasound imaging, the method comprising: imaging placement of a pulse field ablation electrodes at tissue of a patient with an intracardiac echocardiogram catheter; detecting position information for the pulse field ablation device relative to the tissue from the imaging; and displaying a visual cue for alignment of the pulse field ablation device with the tissue, the visual cue based on the position information.

Illustrative Embodiment 2. The method of Illustrative Embodiment 1, wherein imaging comprises imaging by the intracardiac echocardiogram catheter where the pulse field ablation electrodes are at an ostium of a vein.

Illustrative Embodiment 3. The method of any of Illustrative Embodiments 1-2, wherein detecting comprises detecting the pulse field ablation device and detecting the tissue as the position information.

Illustrative Embodiment 4. The method of Illustrative Embodiment 3, wherein detecting comprises detecting an angle of a shaft of the pulse field ablation device relative to the tissue, and wherein the visual cue comprises the angle.

Illustrative Embodiment 5. The method of any of Illustrative Embodiments 3-4, wherein detecting comprises detecting a distance of a part of the pulse field ablation device relative to the tissue, and wherein the visual cue comprises the distance.

Illustrative Embodiment 6. The method of any of Illustrative Embodiments 3-5, wherein detecting comprises detecting a rotational angle of electrodes of the pulse field ablation device relative to the tissue, and wherein the visual cue comprises the rotational angle.

Illustrative Embodiment 7. The method of Illustrative Embodiment 6, further comprising tracking the rotational angle for different ablations by the electrodes on the tissue, and wherein the visual cue comprises a map of the rotational angles of the different ablations.

Illustrative Embodiment 8. The method of any of Illustrative Embodiments 1-7, wherein detecting comprises detecting by an artificial intelligence.

Illustrative Embodiment 9. The method of Illustrative Embodiment 8, wherein the artificial intelligence comprises a multi-task neural network configured by training to output a catheter body and treatment tip of the pulse field ablation device, and wherein detecting comprises detecting the catheter body and the treatment tip by the multi-task neural network.

Illustrative Embodiment 10. The method of any of Illustrative Embodiments 8-9, wherein the artificial intelligence comprises a first machine-learned model configured to identify a plurality of veins and a second machine-learned model configured to detect an ostium of a closest of the veins to the pulse field ablation device, and wherein detecting comprises sequentially detecting the plurality of veins by the first machine-learned model and then detecting the closest of the veins by the second machine-learned model.

Illustrative Embodiment 11. The method of any of Illustrative Embodiments 1-10, wherein detecting comprises detecting electrodes of the pulse field ablation device by a machine-learned model or template matching.

Illustrative Embodiment 12. The method of any of Illustrative Embodiments 1-11, wherein displaying comprises displaying the visual cue as a graphic, highlight, or annotation on ultrasound images from the imaging.

Illustrative Embodiment 13. An ultrasound system for pulse field ablation guidance, the ultrasound system comprising: an intracardiac echocardiogram catheter with a transducer configured for ultrasound scanning from within a cardiac system of a patient; an image processor configured to generate guidance of pulse field ablation from the ultrasound scanning; and a display configured to display the guidance.

Illustrative Embodiment 14. The ultrasound system of Illustrative Embodiment 13, wherein the image processor is configured to generate the guidance as an angle of a pulse field ablation catheter relative to tissue and a distance of a treatment tip of the pulse field ablation catheter to the tissue.

Illustrative Embodiment 15. The ultrasound system of any of Illustrative Embodiments 13-14, wherein the image processor is configured to generate the guidance as a treatment map of electrodes of a pulse field ablation catheter.

Illustrative Embodiment 16. The ultrasound system of any of Illustrative Embodiments 13-15, wherein the image processor is configured to generate the guidance by detection of tissue of the patient and a pulse field ablation catheter.

Illustrative Embodiment 17. A method for guiding pulse field ablation with ultrasound imaging, the method comprising: scanning a pulse field ablation catheter and an ostium of a vein of a patient with an intracardiac echocardiogram catheter; and displaying visual guidance of pulse field ablation catheter deployment in pulmonary vein isolation treatment, the visual guidance generated from the scanning.

Illustrative Embodiment 18. The method of Illustrative Embodiment 17, further comprising generating the visual guidance from detection of the pulse field ablation catheter and the ostium, the visual guidance indicating the pulse field ablation catheter deployment relative to the ostium.

Illustrative Embodiment 19. The method of any of Illustrative Embodiments 17-18, wherein displaying comprises displaying the visual guidance as an angle of a shaft of the pulse field ablation catheter relative to the ostium and as a distance of a treatment tip of the pulse field ablation catheter relative to the ostium.

Illustrative Embodiment 20. The method of any of Illustrative Embodiments 17-19, wherein displaying comprises displaying a map of treatment formed from electrodes of the pulse field ablation catheter during the pulmonary vein isolation treatment with the electrodes at different rotations relative to the ostium.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, including all equivalents, that are intended to define the scope of this invention.

## Claims

1. A method for guiding pulse field ablation with ultrasound imaging, the method comprising:
imaging placement of a pulse field ablation electrodes (453) at tissue of a patient with an intracardiac echocardiogram catheter (400);
detecting position information for the pulse field ablation device (450) relative to the tissue from the imaging; and
displaying a visual cue for alignment of the pulse field ablation device (450) with the tissue, the visual cue based on the position information.

2. The method of claim 1, wherein imaging comprises imaging by the intracardiac echocardiogram catheter (400) where the pulse field ablation electrodes are at an ostium (462) of a vein (460).

3. The method of any of claims 1 - 2, wherein detecting comprises detecting the pulse field ablation device (450) and detecting the tissue as the position information.

4. The method of claim 3, wherein detecting comprises:
detecting an angle of a shaft (456) of the pulse field ablation device (450) relative to the tissue, and wherein the visual cue comprises the angle; and/or
detecting a distance of a part of the pulse field ablation device (450) relative to the tissue, and wherein the visual cue comprises the distance.

5. The method of any of claims 3 - 4, wherein detecting comprises detecting a rotational angle of electrodes of the pulse field ablation device (450) relative to the tissue, and wherein the visual cue comprises the rotational angle,
wherein the method optionally further comprising tracking the rotational angle for different ablations by the electrodes on the tissue, and wherein the visual cue comprises a map of the rotational angles of the different ablations.

6. The method of any of claims 1 - 5, wherein detecting comprises detecting by an artificial intelligence.

7. The method of claim 6,
wherein the artificial intelligence comprises a multi-task neural network configured by training to output a catheter body and treatment tip of the pulse field ablation device (450), and wherein detecting comprises detecting the catheter body and the treatment tip by the multi-task neural network; and/ or
wherein the artificial intelligence comprises a first machine-learned model configured to identify a plurality of veins (460) and a second machine-learned model configured to detect an ostium (462) of a closest of the veins (460) to the pulse field ablation device (450), and wherein detecting comprises sequentially detecting the plurality of veins (460) by the first machine-learned model and then detecting the closest of the veins (460) by the second machine-learned model.

8. The method of any of claims 1 - 7, wherein detecting comprises detecting electrodes of the pulse field ablation device (450) by a machine-learned model or template matching.

9. The method of any of claims 1 - 8, wherein displaying comprises displaying the visual cue as a graphic, highlight, or annotation on ultrasound images from the imaging.

10. An ultrasound system for pulse field ablation guidance, in particular using the method of any one of claims 1 - 9, the ultrasound system comprising:
an intracardiac echocardiogram catheter (400) with a transducer (412) configured for ultrasound scanning from within a cardiac system of a patient;
an image processor (430) configured to generate guidance of pulse field ablation from the ultrasound scanning; and
a display (440) configured to display the guidance.

11. The ultrasound system of claim 10,
wherein the image processor (430) is configured to generate the guidance as an angle of a pulse field ablation catheter (450) relative to tissue and a distance of a treatment tip (454) of the pulse field ablation catheter (450) to the tissue; and/or wherein the image processor (430) is configured to generate the guidance as a treatment map of electrodes (452) of a pulse field ablation catheter (450).

12. The ultrasound system of any of claim 10 - 11, wherein the image processor (430) is configured to generate the guidance by detection of tissue of the patient and a pulse field ablation catheter (450).

13. A method for guiding pulse field ablation with ultrasound imaging, the method comprising:
scanning a pulse field ablation catheter (450) and an ostium (462) of a vein (460) of a patient with an intracardiac echocardiogram catheter (400); and
displaying visual guidance of pulse field ablation catheter (450) deployment in pulmonary vein (460) isolation treatment, the visual guidance generated from the scanning.

14. The method of claim 13, further comprising generating the visual guidance from detection of the pulse field ablation catheter (450) and the ostium (462), the visual guidance indicating the pulse field ablation catheter deployment relative to the ostium (462).

15. The method of any of claims 13 - 14, wherein displaying comprises:
displaying the visual guidance as an angle of a shaft (456) of the pulse field ablation catheter relative to the ostium (462) and as a distance of a treatment tip (454) of the pulse field ablation catheter (450) relative to the ostium (462); and/or
displaying a map of treatment formed from electrodes (452) of the pulse field ablation catheter (450) during the pulmonary vein (460) isolation treatment with the electrodes (452) at different rotations relative to the ostium (462).
